(19) Europäisches Patentamt
European Patent Office
Office européen des brevets

(11) Numéro de publication: **0 140 781**
**A2**

(12) # DEMANDE DE BREVET EUROPEEN

(21) Numéro de dépôt: **84402098.2**

(22) Date de dépôt: **18.10.84**

(51) Int. Cl.[4]: **A 61 K 31/725**
// (A61K31/725, 31:57)

(30) Priorité: **18.10.83 FR 8316583**

(43) Date de publication de la demande: **08.05.85**
**Bulletin 85/19**

(84) Etats contractants désignés: **AT BE CH DE FR GB IT LI LU NL SE**

(71) Demandeur: **DROPIC Société Civile de Gestion de Droit de Propriété Industrielle CHOAY, 10 Avenue Matignon, F-75008 Paris (FR)**

(72) Inventeur: **Choay, Jean, 21, rue Saint-Guillaume, F-75007 Paris (FR)**
Inventeur: **Lormeau, Jean-Claude, 1, rue Joseph Delattre, F-76150 Maromme (FR)**
Inventeur: **Pierpaoli, Walter, Lohwisstrasse, 12, CH-8123 Ermatingen (CH)**

(74) Mandataire: **Peaucelle, Chantal et al, Cabinet Plasseraud 84, rue d'Amsterdam, F-75009 Paris (FR)**

(54) **Compositions renfermant des dérivés d'héparine appropriées notamment au traitement de désordres de la prolifération cellulaire.**

(57) Ces compositions comprennent au moins un composé à activité anti-inflammatoire de type corticoïde et une quantité faible, physiologiquement tolérable associé à au moins un dérivé d'héparine.

EP 0 140 781 A2

"Compositions renfermant des dérivés d'héparine appropriées notamment au traitement de désordres de la prolifération cellulaire".

L'invention a pour objet de nouvelles compositions renfermant des dérivés d'héparine appropriées, notamment, au traitement des désordres de la prolifération cellulaire.

On sait que l'héparine est composée de chaînes d'oligosaccharides et de polysaccharides formés de motifs alternés sucre aminé et acide uronique.

Les motifs sucre aminé présentent une structure du type D-glucosamine et les motifs acide uronique une structure de type acide D-glucuronique ou acide L-iduronique.

L'application de l'héparine comme médicament anticoagulant est également largement connue. Traditionnellement, son activité est exprimée en unités internationales USP. Cette activité est le reflet de ce qu'il est convenu d'appeler une activité anticoagulante globale, dans la mesure où elle paraît affecter un nombre important des facteurs sanguins intervenant au niveau de la coagulation. L'héparine est, en outre, caractérisée par une activité antithrombotique que reflète l'activité particulière à l'égard du facteur X activé (ou activité anti-Xa), cette activité étant exprimée en unités Yin-Wessler (YW) par milligramme. D'une manière générale le rapport du titre YW au titre USP est voisin de 1, les valeurs de ces titres étant d'environ 130 à 160.

On sait également qu'il est possible d'obtenir, à partir de l'héparine, des fractions de bas poids moléculaire, que ce soit par fractionnement ou par dépolymérisation chimique ou enzymatique de l'héparine.

Il est ainsi possible d'isoler des fractions formées essentiellement de chaînes ou fragments d'héparine caractérisés par une activité antithrombotique plus élevée

que l'héparine. Ces chaînes sont dotées d'une affinité pour l'anti-thrombine III ou ATIII d'une activité anti-X au moins de l'ordre de celle de l'héparine ou supérieur et d'une activité USP considérablement déprimée et même pratiquement nulle.

Ces procédés conduisent également à des fractions dépourvues d'affinité pour l'ATIII ou de faible affinité et, par là, ne possédant pratiquement pas d'activité antithrombotique. Le titre USP de ces fractions est également faible, ce qui correspond à des produits pratiquement dépourvus d'activité anticoagulante.

Par synthèse, on a également obtenu des oligosaccharides possédant une structure de même type que certains éléments de structure contenus dans les héparines naturelles ainsi qu'éventuellement les groupes de substitution présents dans les chaînes de ces héparines.

Les différents types de fragments ou chaînes d'héparine évoqués plus haut seront désignés indifféremment ci-après par l'expression "héparine de bas poids moléculaire" (en abréviation héparine de bas PM) ou "oligosaccharide", qui recouvre également leurs sels.

La recherche de moyens permettant d'inhiber et/ou de faire regresser, in vivo, les proliférations cellulaires anormales, en particulier les proliférations de cellules tumorales ont conduit les inventeurs à étudier des compositions renfermant en association ces dérivés d'héparine et des agents anti-inflamatoires du type corticoïde.

L'invention est basée sur la decouverte qu'il est possible de disposer de compositions efficaces au regard des indications ci-dessus et applicables en thérapeutique en associant des dérivés d'héparine avec de faibles quantités d'agent anti-inflammatoire du type corticoïde.

L'invention a donc pour but de fournir de nouvelles compositions applicables en thérapeutique, renfermant des dérivés d'héparine associés à des quantités déterminées

d'agent anti-inflammatoire du type corticoïde.

Elle vise également à fournir de nouvelles compositions pharmaceutiques sous différentes formes d'administration, de grande efficacité pour de traitement de divers désordres de la prolifération cellulaire.

Les compositions de l'invention sont caractérisées en ce qu'elles comprennent au moins un agent à activité anti-inflammatoire du type corticoïde, en une quantité faible, physiologiquement tolérable, associé à une quantité thérapeutiquement efficace d'au moins un dérivé d'héparine.

Par "quantité faible, physiologiquement tolérable", on entend, en ce qui concerne l'agent anti-inflammatoire ci-dessus, une quantité correspondant à une dose d'administration à un patient n'excédant pas environ 5 mg/kg / jour.

Selon un mode de réalisation de l'invention, le dérivé d'héparine est avantageusement constitué par une héparine dans lequelle les motifs glucosamine en position 2 sont substitués par des groupes $-NH_2$ et/ou -NH-acyle, plus spécialement -NH-acétyle, à la place des groupes $-NHSO_3$ rencontrés dans les motifs glucosamine des chaînes naturelles.

Une telle héparine "N-désulfatée" est dépourvue d'activité anti-coagulante globale ainsi que d'activité Yin-Wessler, ce qui présente l'avantage de disposer de compositions dépourvues d'effets au niveau de la coagulation.

Selon un autre mode de réalisation de l'invention, le dérivé d'héparine est formé d'une héparine de bas PM dont la majorité deschaînes ou fragments possède un PM inférieur ou égal à 10 000 daltons, ce qui correspond environ à 30 motifs (sur la base d'un poids moléculaire moyen de 316 daltons pour un motif).

Conformément à l'invention, le PM de ces chaînes ou fragments est plus spécialement inférieur ou égal à environ 8000, ce qui correspond à environ 26 motifs au plus.

Des compositions préférées renferment des chaînes d'héparine formées essentiellement de 4 à 26 motifs ou encore de 6 à 26 motifs environ.

Dans d'autres compositions encore, les chaînes sont formées d'au moins 8 motifs.

Ce nombre de motifs est avantageusement de 6, ou encore 5, ou bien 4 dans d'autres chaînes de compositions de l'invention.

D'une manière avantageuse, l'association de ces dérivés d'héparine avec de faibles quantités de produit à activité anti-inflammatoire du type corticoïde, exerce, notamment, un effet d'inhibition sur le developpement anormal de cellules et/ou le cas échéant, sur l'angiogenèse, en particulier dans le cas des héparines renfermant 5 ou encore 4 motifs.

Des compositions préférées comprennent des héparines de bas PM essentiellement formées de chaînes dépourvues d'affinité pour l'ATIII.

Compte-tenu de l'absence dans ces chaînes d'une séquence capable de se fixer à l'ATIII, ces héparines n'exercent pas ou pratiquement pas d'activité anti-Xa. Leur activité anticoagulante globale est également pratiquement nulle. Les héparines n'interviennent donc pas sur les phénomènes de coagulation ce qui leur confère un grand interêt dans des applications thérapeutiques où le patient présente des problèmes d'hypocoagulation.

Dans d'autres compositions de l'invention, le taux des chaînes dépourvues d'affinité pour l'ATIII représente au moins 95% de l'héparine de bas PM.

Les titres anti-Xa des héparines de bas PM sont alors inférieurs à ceux de l'héparine et leurs titres USP sont très faibles.

On observe, en particulier, des titres anti-Xa inférieurs à 100 et même à 50 environ et des titres USP très faibles à pratiquement nuls.

Le taux de chaînes d'héparine à faible affinité pour l'ATIII ou sans affinité, est moins élevé dans les héparines de bas PM d'autres compositions de l'invention, atteignant 85%, ou encore 75% environ de l'héparine de bas PM.

Les héparines de ces compositions sont alors dotées d'activité anti-Xa plus élevée de l'ordre de 200 à 250 u/mg environ et présentent des rapports YW/USP d'environ au moins 3 à 10.

D'autres compositions renferment des héparines de bas PM dans lesquelles les taux de chaînes ne possédant pas d'affinité pour l'ATIII et de chaînes présentant au contraire une affinité sont sensiblement identiques.

Ce taux est prépondérant dans d'autres compositions de l'invention.

Ce taux peut être de l'ordre de 75% de l'héparine de bas PM et même atteindre 100%.

Les héparines de bas PM correspondantes sont dotées d'une activité anti-Xa élevée, d'au moins 300 u/mg, notamment entre 300 et 500, et pouvant atteindre 2000 à 3000 u/mg et d'un titre USP très faible à pratiquement nul.

Dans un groupe de compositions de l'invention, les chaînes d'héparine sont caractérisées par un enchaînement de motifs dont la structure et éventuellement une partie ou la totalité des groupes de substitution, en ce qui concerne leur nature et leur position, correspondent à ceux des chaînes d'héparine naturelle.

Ces chaînes sont avantageusement obtenues selon le procédé de synthèse décrit, notamment, dans les demandes FR 81 08412 du 28.04.1981, 82 180003 du 27.10.1982 et 84 07589 du 16.05.1984 au nom de Demanderesse.

Dans un autre groupe, au moins un motif structural des héparines de bas PM, plus spécialement en début ou en fin de chaîne, se trouve chimiquement modifié par rapport au motif correspondant des chaînes naturelles. Cette modification résulte plus spécialement du procédé mis en oeuvre pour leur obtention.

Ainsi, la dépolymérisation nitreuse de l'héparine conduit à une coupure de la liaison entre deux motifs respectivement de structure N-sulfate D-glucosamine et acide L-iduronique, le remaniement du motif N-sulfate glucosamine conduisant à un motif de structure 2,5-anhydromanno.

Des oligosaccharides dont les extrémités de chaînes correspondent à de telles structures sont décrits, notamment, dans la deuxième demande de certificat d'addition No 80 06282 du 20.03.1980 au brevet principal 78 31357 du 6.11.1978 de la Demanderesse. Dans cette demande de certificat d'addition, on décrit également la transformation de la structure 2,5-anhydromannose, au cours d'étapes ultérieures, en une structure 2,5-anhydro-mannitol ou acide 2,5-anhydromannonique. Ces structures correspondent à des produits particulièrement intéressants, notamment, en raison de leur stabilité. On rappelle que leur titre YW est voisin de 200 u/mg et leur rapport YW/USP d'environ 10.

Des oligosaccharides de ce type sont également obtenus selon le procédé de dépolymérisation autorégulée de l'héparine décrit dans la demande FR 81 07283 du 10.04.1981 au nom de la Demanderesse.

Des oligosaccharides de plus faible poids moléculaire, renfermant au plus 8 motifs sont décrits dans la demande EP 80 401425.6 du 6.10.1980 également au nom de la Demanderesse.

Parmi les demandes de brevet de l'art antérieur faisant état d'héparine de bas poids moléculaire, possédant une activité anti-Xa supérieure à celle de l'héparine dont les chaînes sont terminées par un groupe de structure 2,5-anhydromannose, on citera les demandes EP 80 850002.9 du 7.01.1981 et 81 8501154.6 du 10.09.1981 au nom de KABI AB et la demande EP 81 301 825.6 du 24.04.1981 au nom de RIKER Laboratories inc.

Chez d'autres héparines de bas PM mises en oeuvre dans les compositions de l'invention, le motif de début de chaîne peut être insaturé par suite d'une coupure enzymatique par exemple, avec une héparinase.

Ce motif insaturé, qui se trouve donc à l'extrémité non réductrice de la chaîne correspond à un motif acide iduronique insaturé.

La structure des motifs terminaux, c'est-à-dire, à l'extrémité réductrice n'est pas, en revanche, modifiée et correspond à celle rencontrée d'une manière générale dans les chaînes naturelles d'héparine. L'enchaînement disaccharidique de l'extrémité réductrice présente une structure du type [acide 2-O-sulfate L-iduronique]-[N-sulfate 6-O-sulfate D-glucosamine].

Des oligosaccharides de ce type comprennent certains des oligosaccharides décrits dans la demande EP 80 401426.6 rappellée ci-dessus et les hexa-saccharides définis notamment dans la demande FR 81 08604 du 29.04.1981 au nom de la Demanderesse. L'activité anti-Xa de ces oligosaccharides est élevée et atteint des valeurs d'au moins 2000 à 3000 unités YW par mg. Leurs titres USP sont faibles à pratiquement nuls.

Des oligosaccharides de 2000 à 10 000 daltons environ possédant également un motif insaturé en début de chaîne sont décrits dans la demande EP 81 400728.2 au nom de Pharmin industrie du 8.05.1981.

Des héparines de bas poids moléculaire sont, en outre, décrites dans les brevets FR 78 23499 du 9.08.1978 et 81 01508 du 27.02.1981, respectivement au nom d'Hépar Chimie s.a. .Hépar Industries inc.

Dans une autre variante de réalisation de l'invention, les oligosaccharides peuvent être formés de chaînes correspondant aux chaînes naturelles de bas poids moléculaire de l'héparine.

Dans le brevet FR 78 31357 du 6.11.1978 et le premier certificat d'addition No 79 18873 du 20.07.1979 au brevet ci-dessus, au nom de la Demanderesse, on décrit des héparines de ce type, de poids moléculaire inférieur ou égal à 10 000, dotées d'un rapport du titre

YW au titre USP d'au moins 3 avec un titre YW voisin de celui de l'héparine et pouvant être légèrement inférieur.

Dans les compositions de ces différents modes de réalisation de l'invention, l'agent anti-inflammatoire du type corticoïde est avantageusement choisi parmi la cortisone, l'hydrocortisone, l'isomère 11$\alpha$ de l'hydrocortisone ou cortisol, la prednisone, la prednisolone, la méthylprednisolone, la triamcinolone, la paraméthasone, la bétaméthasone, la dexaméthasone, leurs dérivés, notamment leurs sels, ou analogues.

D'une manière préférée, les compositions de l'invention renferment en association, un corticoïde tel qu'évoqué ci-dessus plus spécialement de la cortisone, selon une quantité telle que la dose administrée ne dépasse pas 5mg/kg/jour et de l'héparine "désulfatée" et dans laquelle la position 2 des motifs glucosamine est occupée par un groupe $-NH_2$ ou -NH-acyle, plus spécialement -NH-acétyle.

D'autres compositions préférées renferment en association un corticoïde comme indiqué ci-dessus, avantageusement de la cortisone, avec des héparines de bas PM, plus spécialement des héparines formées de chaînes comportant de 4 à 26 motifs et terminées par des groupes de structure 2,5-anhydromanno ou encore comportant de 6 à 26 motifs et correspondant aux chaînes naturelles d'héparine telles qu'obtenues par un fractionnement du type du fractionnement alcoolique.

Dans d'autres compositions avantageuses, il s'agit d'associations d'un corticoïde, avantageusement de cortisone, avec une héparine de bas PM formée de chaînes renfermant 8 ou plus de 8 motifs.

Dans ces chaînes, les motifs terminaux présentent plus spécialement une structure 2,5-anhydromanno et l'extrémité non réductrice n'est pas modifiée par rapport aux chaînes naturelles.

D'autres compositions, particulièrement préférées en raison plus spécialement de leurs propriétés inhi-

bitrices vis-à-vis de l'angiogénèse comprennent un corticoïde, ______________ associé à une héparine de bas PM ne renfermant pas plusde 5 motifs. Une héparine de ce type est avantageusement constituée par un pentasaccharide obtenu par voie de synthèse, en particulier, par un pentasaccharide possédant la structure du type DEFGH comme décrit dans la demande FR 82 180003 dont question ci-dessus.

Un pentasaccharide de type DEFGH spécialement préféré correspond au composé No 50 de cette demande de brevet. Ce composé, dans lequel le motif F possède un groupe sulfate en position 3 est doté d'une activité anti-Xa atteignant 3000 u/mg et d'un titre USP pratiquement nul.

Dans un autre pentasaccharide de ce type, le motif F ne comporte pas de groupe sulfate en position 3 mais comprend un groupe -OH. Ce composé qui est décrit dans la demande FR 84 07589 mentionnée plus haut est dépourvu d'activité anticoagulante. Ce composé présente donc l'avantage, lors de sa mise en oeuvre dans les applications thérapeutiques envisagées dans l'invention, de ne pas exercer d'effet au niveau des phénomènes mis en jeu dans la coagulation sanguine.

L'étude pharmacologique de ces associations de corticoïdes et de dérivés d'héparine a mis en évidence leur efficience lors de multiplications cellulaires anormales. On constate d'une manière générale un effet d'inhibition et/ou de regression des proliférations anormales.

En particulier, ces associations sont capables d'inhiber le développement de tumeurs, notamment du type mélanomes ou lymphosarcomes et permettent avantageusement de faire régresser ces tumeurs.

Cet effet inhibiteur s'exerce également au niveau de l'angiogénèse en empêchant le développement de la vascularisation et par là, le cas échéant, de tumeurs.

Selon un aspect de grant intérêt, cet effet s'exerce in vivo avec de faibles doses, tolérables par l'organisme, d'agent anti-inflammatoire du type corticoïde, ce qui permet une utilisation de ces associations en thérapeutique sans les effets secondaires importants liés à l'utilisation intensive de corticoïdes.

L'invention vise donc également ces nouvelles compositions pharmaceutiques renfermant, en combinaison avec un véhicule pharmaceutiquement acceptable, une quantité efficace d'au moins un dérivé d'héparine tel que défini ci-dessus, associé à une dose faible physiologiquement tolérable de corticoïde.

Elle est plus particulièrement relative à des compositions pharmaceutiques dépourvues de substances pyrogènes, contenant une quantité efficace de principes actifs en combinaison avec des excipients pharmaceutiques.

Ces compositions renferment avantageusement des quantités de corticoïde telles que la dose administrée au patient ne dépasse pas 5 mg / kg par jour. De préférence, cette quantité est telle que la dose administrée est inférieure à 2 mg/kg. par jour.

Elle concerne également les compositions dans lesquelles le véhicule pharmaceutique est approprié pour l'administration par voie orale. Des formes d'administration de l'invention appropriées pour l'administration par voie orale peuvent être avantageusement des gélules gastrorésistantes, des comprimés ou tablettes, des pilules, ou encore se présenter sous forme de liposomes ou de solutions buvables. Ces préparations renferment avantageusement de 50 mg à 5 g de composition par unité de prise, de préférence 200 à 250 mg pour les gélules, tablettes et pilules, et 0,5 à 5 g pour les solutés buvables.

En conséquence, les formes d'administration dépendent essentiellement de la dose à administrer.

Pour l'administration de doses plus élevées, les solutés buvables pourront être d'une utilisation plus pratique.

D'autres compositions pharmaceutiques comprennent ces compositions en association avec les excipients appropriés pour l'administration par voie rectale.

D'autres formes d'administration de l'invention sont constituées par des aérosols ou des pommades.

L'invention concerne également des compositions pharmaceutiques injectables, stériles ou stérilisables pour l'administration tant par voie intraveineuse qu'intramusculaire ou sous-cutanée.

Ces solutions renferment avantageusement de 50 à 250mg/ml composition, de préférence de 100 à 150 mg/ml lorsque ces solutions sont destinées à l'injection par voie sous-cutanée. Elles peuvent contenir, par exemple, de 25 à 250, notamment de 150mg/ml de composition lorsqu'elles sont destinées à l'injection par voie intraveineuse ou par perfusion.

Avantageusement, de telles préparations pharmaceutiques sont présentées sous la forme de seringues non récupérables, prêtes à l'emploi.

Les compositions de l'invention sont plus spécialement appropriées pour lutter contre le vieillissement des tissus ou des manifestations de type dégénératif telles que les alopécies.

Afin d'illustrer l'invention, on indique, ci-après, un exemple de posologie utilisable chez l'homme : cette posologie comprend, par exemple, l'administration au patient de 50mg à 150mg par voie sous-cutanée, une à trois fois par jour, selon le degré de développement des proliférations cellulaires anormales, ou de 150mg /24 heures,

par voie intraveineuse, en administrations discontinues à intervalles réguliers, ou continues par perfusion, ou encore de 150 mg (trois fois par semaine) par voie intramusculaire ou sous-cutanée. Ces doses peuvent être naturellement ajustées pour chaque patient en fonction des résultats et des analyses effectuées auparavant, la nature des affections dont il souffre et, d'une manière générale, son état de santé et l'état de coagulation.

Comme indiqué ci-dessus, toute une gamme de dérivés d'héparine, plus spécialement d'héparines de bas PM, est disponible, qu'il s'agisse de fractions ou fragments obtenus à partir d'héparines naturelles ou d'oligosaccharides obtenus par synthèse. Selon la nature des chaînes d'héparine, l'activité anticoagulante peut être importante à quasiment nulle.

Il est donc possible de choisir parmi ces différentes fractions celles qui seront les plus compatibles avec l'état de la coagulation, éventuellement d'hypocoagulation du patient à traiter. Il pourra être utile de choisir, parmi ces fractions d'héparine, celles dont les activités USP sont les plus réduites, voire nulles, lorsque le traitement s'adressera à un patient sujet à de sérieux risques d'hypocoagulation.

L'invention concerne encore les réactifs biologiques dont les principes actifs sont constitués par des compositions définies plus haut, ces réactifs biologiques pouvant être utilisés comme références ou étalons dans des études des éventuelles propriétés anti-angiogénèse d'autres substances, notamment dans des tests mettant en jeu les techniques décrites dans les exemples suivants .

Des caractéristiques supplémentaires de l'invention apparaîtront encore au cours de la description qui suit d'essais biologiques effectués avec des dérivés d'héparine constituant les principes actifs des compositions pharmaceutiques de de l'invention.

Dans les exemples 1 et 2 qui suivent, on rapporte les résultats de l'étude de l'activité antitumorale de quatre compositions comprenant, en association, de la cortisone et un dérivé d'héparine a, b, c ou d, Ces dérivés répondent aux caractéristiques données dans le tableau 1 suivant :

| | dérivé d'héparine | méthode d'obtention | nombre de motifs de la majorité des chaînes | titre anti-Xa | titre USP |
|---|---|---|---|---|---|
| a : | héparine N-désulfaté N-acétylée | désulfatation selon K.Nagasawa et al * acétylation selon Cifonelli** | - | 0 | 0 |
| b : | héparine formée essentiellement de chaînes naturelles de PM moyen de 4500 | par extraction alcoolique à partir d'héparine selon le procédé du brevet FR 7831357 déjà cité | 6 - 26 | 200 | 50 |
| c : | fragments d'héparine dépolymérisée à terminaison 2,5 anhydro-mannitol | par dépolymérisation nitreuse d'héparine selon le 2ème certificat d'addition 80 06282 déjà cité, suivie d'une étape d'hydrogénation. | 4 - 26 | 250 | 25 |
| d : | héparine de bas PM | par fractionnement sur Séphadex G50 du produit c | $\geqslant$ 8 | 360 | 51 |

* dans Carb.Res.58 p.47-55,1977

** dans Carb.Res. 37,p. 145-159 (1974)

Exemple 1 : Action sur un mélanome de type B16 transplanté sur la souris.

Modèle expérimental :

On utilise une suspension de cellules de mélanome fraîches à la concentration standard. Après culture, la suspension cellulaire est lavée, puis centrifugée. Le culot cellulaire est repris dans du milieu M 199 à raison de 1 ml du culot pour 4 ml de milieu.

0,2 ml de cette suspension est inoculée par voie sous-cutanée sur le dos de la souris.

Le test s'effectue sur des souris C 57 BL/6. La tumeur est syngénéique pour cette espèce et ne se développe pas dans d'autres espèces.

Les animaux sont répartis en groupes de 10. Un groupe sert de contrôle ; le ou les autres groupes sont traités avec les produits à tester.

Au jour J0, tous les animaux reçoivent une injection de cellules de mélanome, 0,2 ml d'une suspension à la concentration standard.

Au bout de 6 à 7 jours, les tumeurs atteignent un volume suffisant pour pouvoir être reconnues à la palpation.

Le premier groupe reçoit alors 2 injections quotidiennes intrapéritonéales du produit à tester, chaque injection contenant de 150 à 400 µg de dérivé d'héparine et 50µg de cortisone dans 0,5 à 0,7ml de soluté chloruré isotonique.

Le deuxième groupe reçoit le même volume de soluté chloruré isotonique.

Le traitement est poursuivi pendant 5 jours et les animaux sont alors sacrifiés.

Les tumeurs sont prélevées et pesées et l'on compare le poids des tumeurs dans les deux groupes.

Les résultats sont exprimés en pourcentage d'inhibition de la croissance chez les animaux traités par rapport aux animaux contrôle n'ayant reçu que le soluté chloruré isotonique.

RESULTATS

| | Produit | Dose quotidienne par souris | Poids tumeur | % d'inhibition |
|---|---|---|---|---|
| Exp. N° 1 | témoins | - | 1456 ± 777 | 0 |
| | a | | | 46% |
| | b | 2 x 150 µg | 1488 ± 1015 | 0(i.p);29(p.o) |
| | b | 2 x 300 µg | 1359 ± 692 | 20 |
| | c | 2 x 400 µg | 1054 ± 615 | 27 à 38 |
| | d | | | 12 |

Ces résultats mettent en évidence l'action favorable des principes actifs des compositions de l'invention sur l'inhibition et la régression des tumeurs.

EXEMPLE 2 : Action de c sur le lymphosarcome 18

Modèle expérimental :

On utilise des cellules de lymphosarcomes provenant de donneurs leucémiques. On utilise des souris C 57 BL/6. Les animaux reçoivent par voie intrapéritonéale 20 x $10^6$ cellules de lymphosarcome au jour J0.

Le traitement commence au jour J0 et est poursuivi pendant un mois à raison de deux injections/de 150
par animal

à 500 $\mu g$ du dérivé C et 60 $\mu g$ d'hydrocortisone, par voie intrapéritonéale, dans un volume de 0,5 à 0,7 ml.

Au bout d'un mois, les animaux sont sacrifiés et autopsiés et l'on compte le nombre de sarcomes sur thymus, le fois, la rate et les ganglions lymphatiques.

RESULTATS

Deux groupes de 6 animaux ont été utilisés, l'un servant de contrôle. Le groupe traité reçoit journellement deux injections de 500 $\mu g$ du produit 0,5 ml de soluté chloruré isotonique. Le groupe contrôle reçoit le même volume du soluté chloruré isotonique seul. Parmi le groupe traité, deux animaux sont morts d'hémorragie au cours de l'expérience. L'un d'entre eux, à l'autopsie, ne présentait aucun sarcome.

Les résultats figurent dans le tableau suivant. Ils sont donnés par animal survivant. Les croix représentent le nombre de sarcomes. Le tableau indique également les organes sur lesquels les comptages ont été effectués.

| Résultats | Témoins | Animaux traités |
|---|---|---|
| thymus | * | *** |
| | *** | ** |
| | * | - |
| | *** | - |
| foie | ** | - |
| | * | * |
| | ** | - |
| | *** | - |
| rate | ** | * |
| | * | ** |
| | *** | - |
| | *** | - |
| ganglions lymphatiques | **** | *** |
| | ** | **** |
| | *** | * |
| | **** | - |

On constate donc une réduction globale des nombres à la fois de sarcomes et des animaux affectés, lorsque ceux-ci ont été traités, vis-à-vis des résultats observés chez les animaux témoins.

Dans l'exemple 3 qui suit, on indique l'activité de compositions de l'invention sur l'inhibition de la néoangiogénèse :

EXEMPLE : Etude de l'activité anti-néoangiogénèse de compositions de l'invention

Les essais in vitro relatifs à l'activité anti-angiogénèse ont été réalisés sur la membrane chorioallantoïque d'embryons de poulets 6 jours en culture dans des récipients de pétri.

On forme des pastilles en mélangeant 50 µg d'hydrocortisone à 10 µl de méthylcellulose, puis on ajoute le produit à tester selon des doses allant de 6 µg à 100 µg . Les pastilles formées sont séchées puis placées sur la membrane chorioallantoïque à laquelle elles adhèrent.

Pour chaque dose de produit actif on utilise 4 oeufs.

Après 48 heures d'incubation, on examine la présence des zones avasculaires.

On rapporte dans le tableau I, ci-après, les résultats obtenus avec trois compositions de l'invention dans lesquelles les héparines de bas poids moléculaire respectivement α, β, γ, présentent les caractéristiques suivantes :

α : pentasaccharide de structure DEFGH de formule :

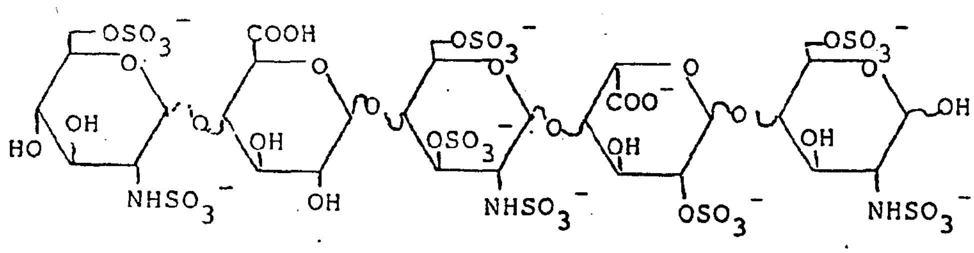

activité anti-Xa de 3000 u/mg

β : pentasaccharide de structure DEFGH comme indiqué ci-dessus mais avec un motif F comportant un groupe -OH en position 3 -

γ : tétrasaccharide de structure DEFG, selon les motifs ci-dessus (F comportant un groupe $-OSO_3^-$ en position 3).

TABLEAU 2

| Héparine de bas PM de la composition | Activité anti-angiogénèse |
|---|---|
| α | actif à 12 µg |
| β | actif à 12 µg |
| γ | actif à 25 µg |

REVENDICATIONS

1.- Composition dotée notamment de propriétés d'inhibition et/ou de régression vis-à-vis de proliférations cellulaires anormales, caractérisée en ce qu'elle comprend au moins un composé à activité anti-inflammatoire de type corticoïde en une quantité faible, physiologiquement tolérable, associé à une quantité thérapeutiquement efficace d'au moins un dérivé d'héparine.

2.- Composition selon la revendication 1, caractérisée en ce que le dérivé d'héparine est constitué par une héparine danslaquelle la majeure partie des motifs glucosamine comprend en position 2 un groupe $-NH_2$ ou NH-acyle, notamment NH-acétyle, à la place des groupes $-NHSO_3^-$ présents dans les chaînes naturelles d'héparine.

3.- Composition selon la revendication 1, caractérisée en ce que le dérivé d'héparine est formé de chaînes ou fragments dont la majeure partie possède un poids moléculaire inférieur ou égal à 10 000 environ, plus spécialement inférieur ou égal à environ 8000.

4.- Composition selon la revendication 3, caractérisée en ce que la majeure partie des chaînes ou fragments d'héparine comprend de 4 à 26 motifs, ou encore de 6 à 26 motifs, ou au moins 8 motifs, le cas échéant 5 ou 4 motifs.

5.- Composition selon l'une quelconque des revendications 1 à 4, caractérisée en ce qu'elle ne renferme pas ou pratiquement pas de chaînes ayant une affinité pour l'AT-III.

6.- Composition selon l'une quelconque des revendications 1 à 4, caractérisée en ce que le taux de chaînes dépourvues d'affinité pour l'ATIII représente au moins 95% de l'héparine de bas PM.

7.- Composition selon la revendication 5 ou 6, caractérisée en ce qu'elle est dépourvue ou pratiquement dépourvue d'activité anti-Xa.

8.- Composition selon l'une quelconque des revendications 1 à 4, caractérisée en ce que le taux de chaînes à faible affinité ou dépourvues d'affinité pour l'ATIII représente 85% ou encore 75% de l'héparine de bas PM.

9.- Composition selon l'une quelconque des revendications 1 à 4, caractérisée en ce qu'elle renferme au moins 50% environ de chaînes ayant une affinité pour l'ATIII, ou encore au moins 75% environ ou pratiquement 100% de chaînes ayant une affinité pour l'ATIII.

10.- Composition selon la revendication 5 ou 6, caractérisée en ce que les chaînes d'héparine possèdent des titres anti-Xa inférieurs à 100 et même 50 environ et des titres USP très faibles à pratiquement nuls.

11.- Composition selon la revendication 8, caractérisée en ce que les chaînes d'héparine de bas PM possèdent un titre YW de l'ordre de 250 u/mg environ et un rapport YW/USP d'au moins environ 10, ou encore un titre YW voisin de celui de l'héparine et un rapport YW/USP d'au moins environ 3.

12.- Composition selon la revendication 9, caractérisée en ce que les chaînes d'héparine de bas PM possèdent une activité anti-Xa supérieure à 300, notamment entre 300 et 500, qui peut atteindre 2000 u/mg, et même 3000 u/mg.

13.- Composition selon l'une quelconque des revendications précédentes, caractérisée en ce que les chaînes d'héparine sont formées d'un enchaînement de motifs dont

la structure et éventuellement une partie ou la totalité des groupes de substitution correspondent à ceux des chaînes d'héparine.

14.- Composition selon l'une quelconque des revendications 1 à 12, caractérisée en ce qu'au moins un motif structural, plus spécialement en début et/ou en fin de chaîne, pour les héparines de bas PM, est ou sont chimiquement modifié (s) et notamment que les chaînes d'héparine de bas PM comportent en fin de chaîne un groupe de structure 2,5-anhydromanno choisi de préférence parmi les groupes 2,5-anhydromannose, 2,5-anhydromannitol et acide 2,5-anhydromannonique, ou encore que les chaînes d'héparine de bas PM comportent en début de chaîne un motif iduronique insaturé.

15.- Composition selon la revendication 13, caractérisée en ce que les héparines de bas PM correspondent aux chaînes naturelles d'héparine et possèdent un titre anti-Xa voisin de celui de l'héparine et un rapport du titre anti-Xa au titreUSP d'au moins 3.

16.- Composition selon la revendication 13, caractérisée en ce que les héparines de bas PM sont obtenues par voie de synthèse et renferment 5 motifs répondant à un enchaînement de structure DEFGH comportant en position 3, un groupe $-OSO_3^-$ ou un groupe -OH, ou que ces héparines renferment 4 motifs répondant à un enchaînement de structure DEFG, le motif F comportant en position 3 un groupe $-OSO_3^-$.

17.- Composition selon l'une quelconque des revendications précédentes caractérisée en ce que l'agent de type corticoïde est choisi parmi la cortisone, l'hydrocortisone l'isomère 11$\alpha$ de l'hydrocortisone ou cortisol, la prednisone,

la prednisolone, la méthylprednisolone, la triamcinolone, la paraméthasone, la bétaméthasone, la dexaméthasone, ou analogue.

18.- Composition pharmaceutique, caractérisée en ce qu'elle renferme, en association avec un véhicule pharmaceutique, un dérivé d'héparine et un agent de type corticoïde, comme défini dans l'une quelconque des revendications 1 à17.

19.- Réactif biologique utilisable comme référence ou étalon dans la réalisation de mesures comparatives des activités antitumorales, caractérisé par le fait que le principe actif de ce réactif est constitué par une composition conforme à l'une quelconque des revendications 1 à 17.